Europäisches Patentamt

European Patent Office

Office européen des brevets

(11) Publication number: **0 355 996**
**A2**

# EUROPEAN PATENT APPLICATION

(21) Application number: **89307375.9**

(22) Date of filing: **20.07.89**

(51) Int. Cl.⁴: **A61B 17/22 , A61B 1/06**

(30) Priority: **21.07.88 US 222638**

(43) Date of publication of application:
**28.02.90 Bulletin 90/09**

(84) Designated Contracting States:
**DE FR GB IT NL**

(71) Applicant: **ADVANCED INTERVENTIONAL
SYSTEMS, INC.**
**3180 Pullman Street**
**Costa Mesa California 92626(US)**

(72) Inventor: **Goldenberg, Tsvi**
**5052 Berean Lane**
**Irvine California 92715(US)**
Inventor: **Shaolian, Samuel M.**
**104 Sequoia Tree Lane**
**Irvine California 92715(US)**

(74) Representative: **Driver, Virginia Rozanne et al**
**Page White & Farrer 54 Doughty Street**
**London WC1N 2LS(GB)**

(54) Guidance and delivery system for high-energy pulsed laser light and endoscope.

(57) A guidewire (1) for a catheter comprises a wire coil (2) having a distal end and a optical fiber (5) having a distal end and extending axially through the center of the wire coil. The distal end of the optical fiber is bonded to the distal end of the wire coil.

FIG. 1

# GUIDANCE AND DELIVERY SYSTEM FOR HIGH-ENERGY PULSED LASER LIGHT AND ENDOSCOPE

## BACKGROUND OF THE INVENTION

### 1. Field of the Invention

The present invention relates to a guidewire for use in performing Percutaneous Transluminal Angioplasty and related procedures for revascularizing an occluded artery.

### 2. Description of Related Art

The use of laser energy to ablate atherosclerotic plaque that forms an obstruction in a blood vessel is presently being investigated as a viable alternative to coronary bypass surgery. This procedure, known as angioplasty, essentially involves insertion of a fiberoptic waveguide into the vessel, and the conduction of laser energy through the waveguide to direct it at the plaque once the distal end of the waveguide is positioned adjacent the obstruction. To enable the physician to ascertain the location of the waveguide as it is being moved through the vessel, additional waveguides for providing a source of illuminating light and for conducting the image from inside the vessel to the physician are fed together with the laser waveguide. Typically, the three waveguides are encapsulated within a catheter.

Most of the experimentation and testing that has been done in this area has utilized continuous wave laser energy, such as that produced by Argon Ion, Nd:YAG or Carbon Dioxide lasers. The light produced by this type of laser is at a relatively low energy level. Ablation of the obstruction is achieved with these types of lasers by heating the plaque with constant laser power over a period of time until the temperature is great enough to destroy it.

While the use of continuous wave laser energy has been found to be sufficient to ablate an obstruction, it is not without its drawbacks. Most significantly, the destruction of the obstruction is uncontrolled and may be accompanied by thermal injury to the vessel walls immediately adjacent the obstruction. In an effort to avoid such thermal injury and to provide better control of the tissue removal, the use of a different, higher level form of laser energy having a wavelength in the ultra-violet range (40-400 nanometers) has been suggested. See, for example, International Patent Application PCT/US84/02000, published June 20, 1985. One example of a laser for producing this higher level energy is known as the Excimer laser, which employs a laser medium such as argon-chloride having a wavelength of 193 nanometers, krypton-chloride (222 nm), krypton-fluoride (248 nm), xenon-chloride (308 nm) or xenon-fluorine (351 nm). The light produced by this type of laser appears in short bursts or pulses that typically last in the range of ten to hundreds of nano-seconds and have a high peak energy level, for example as much as 200 mJ. Although the destruction mechanism involving this form of energy is not completely understood, it has been observed that each single pulse of the Excimer laser produces an incision which destroys the target tissue without accompanying thermal injury to the surrounding area. This result has been theorized to be due to either or both of two phenomena. The delivery of the short duration, high energy pulses may vaporize the material so rapidly that heat transfer to the non-irradiated adjacent tissue is minimal. Alternatively, or in addition, ultraviolet photons absorbed in the organic material might disrupt molecular bonds to remove tissue by photochemical rather than thermal mechanisms.

While the high peak energy provided by Excimer and other pulsed lasers has been shown to provide improved results with regard to the ablation of atherosclerotic plaque, this characteristic of the energy also presents a serious practical problem. Typically, to couple a large-diameter laser beam into a smaller diameter fiber, the fiber input end is ground and polished to an optical grade flat surface. Residual impurities from the polishing compound and small scratches on the surface absorb the laser energy. These small imperfections result in localized expansion at the surface of the fiber when the laser energy is absorbed. The high-energy Excimer laser pulses contribute to high shear stresses which destroy the integrity of the fiber surface. Continued application of the laser energy causes a deep crater to be formed inside the fiber. Thus, it is not possible to deliver a laser pulse having sufficient energy to ablate tissue in vivo using a conventional system designed for continuous wave laser energy.

This problem associated with the delivery of high energy laser pulses is particularly exacerbated in the field of coronary angioplasty because of the small diameter optical fibers that must be used. For example, a coronary artery typically has an internal diameter of two millimeters or less. Accordingly, the total external diameter of the angioplasty system must be below two millimeters. If the system is composed of three separate optical fibers arranged adjacent one another, it will be appreciated that each individual fiber must be quite small in cross-sectional area.

A critical parameter with regard to the destruction of an optical fiber is the density of the energy that is presented to the end of the fiber. In order to successfully deliver the laser energy, the energy density must be maintained below the destruction threshold of the fiber. Thus, it will be appreciated that fibers having a small cross-sectional area, such as those used in angioplasty, can conduct only a limited amount of energy if the density level is maintained below the threshold value. This limited amount of energy may not be sufficient to efficiently ablate the obstructing tissue or plaque without thermal damage.

Even if the energy density is quite high, the small beam that results from the small diameter fiber may not have a sufficiently large target area that effective ablation of the lesion results. Only a small fragment of the lesion might be ablated, and thus not provide adequate relief from the blockage.

In Percutaneous Transluminal Angioplasty (PTA) and Percutaneous Transluminal Coronary Artery (PTCA) a guidewire is directed through an artery past the occlusion in the artery. The guidewire is typically composed of a flexible coiled wire having a safety wire at the tip thereof and a coat of plastic material having a low coefficient of friction, such as teflon, provided over the coiled wire. A balloon catheter is then conducted along the guidewire to the point of occlusion, where the balloon catheter is then inflated to predetermined pressures for predetermined intervals in order to revascularize the artery. Alternatively, instead of using a balloon catheter, a laser catheter could be used to ablate the occlusion with laser energy. However, in the case of a total occlusion, PTA and PTCA cannot be performed because the guidewire is not able to be inserted past the occlusion.

## OBJECTS AND BRIEF STATEMENT OF THE INVENTION

Accordingly, it is a general object of the invention to provide a novel system for delivery high energy pulsed laser light using an optical waveguide.

It is a more specific object of the invention to provide such a delivery system that is particularly well suited to deliver ultraviolet laser energy in vivo for the ablation of atherosclerotic plaque. In this regard, it is a particular object of the present invention to provide a highly efficient waveguide for use in such a delivery system.

It is yet another object of the present invention to provide such a delivery system that is adapted to minimize the likelihood of perforating or otherwise damaging a blood vessel in which the system is being used.

It is a further object of the present invention to provide such a system that includes a guide for facilitating the manoeuvering of the optical waveguides through the blood vessel in which the system is being used.

It is another object of the present invention to provide a guidewire that may be inserted through an artery beyond a total occlusion.

It is yet another object of the present invention to provide a guidewire that includes laser means for ablating an opening in an occlusion in an artery.

Briefly, one aspect of a delivery system embodying the present invention relates to a guidance system that facilitates guiding an optical fiber system through a blood vessel. In a preferred form, the guidance system comprises a guidewire that is inserted into the blood vessel wherein an optical fiber capable of transmitting laser energy is disposed within said guidewire. The distal end of the optical fiber is bonded within the distal end of the guidewire.

The invention also provides a guidewire for a catheter, comprising:
a hollow, flexible means for guiding a catheter through a lumen; and
an optical fiber disposed within said guiding means.

The invention further provides a guidewire for a catheter, comprising:
a hollow, flexible means for guiding a catheter through a lumen;
an angioscope disposed within said guiding means.

The invention further provides a catheter, comprising:
an outer tube;
an angioplasty instrument disposed within the outer tube;
guidewire means disposed within the outer tube for guiding the outer tube through a lumen, said guidewire means including a flexible, hollow sheath and an optical fiber disposed within said sheath.

Further features of the present invention and preferred modes for implementing them will become apparent from the following detailed description of preferred embodiments of the invention illustrated in the accompanying drawings.

## BRIEF DESCRIPTION OF THE DRAWINGS

Figure 1 is a perspective view of the guidewire of the present invention;

Figure 2 is a perspective view of a guidewire and a sleeve used to control movement of the waveguide;

Figure 3 is a schematic diagram of a laser and image delivery system that can be used for angioplasty;

Figure 4 is a cross-sectional side view of a delivery system for high energy Excimer laser light utilizing a funnel-shaped energy coupler;

Figure 5 is a cross-sectional side view of a second embodiment of an energy coupler;

Figure 6A is a side view, partly in section, of a third embodiment of an energy coupler;

Figure 6B is an enlarged view of a portion of Figure 6A, illustrating the principle of operation of this embodiment;

Figure 7 is a perspective view of an energy coupler;

Figure 8 is a perspective view of a multilumen, multifiber catheter;

Figure 9 is a cross-sectional end view of the distal portion of the catheter of Figure 8;

Figure 10 is an enlarged cross-sectional side view of the catheter of Figure 8;

Figure 11 is a perspective view of an alternative embodiment of a multifiber catheter;

Figure 12 is an end view of an alternative embodiment of a catheter that employs balloons for guiding a fiber optic waveguide;

Figure 13 is a perspective view of an alternative embodiment of a multilumen catheter;

Figure 14 is a perspective view of another alternative embodiment of a multilumen catheter;

Figure 15 is a perspective view of another alternative embodiment of a catheter;

Figure 16 is a perspective view of another alternative embodiment of a catheter;

Figure 17 is a perspective view of an angioscope according to the present invention;

Figure 18 is a cross-sectional view taken along line 18-18 of Figure 17; and

Figure 19 is a graph showing pulse construction.

## DESCRIPTION OF THE ILLUSTRATED EMBODIMENTS

In Percutaneous Transluminal Angioplasty (PTA) and in Percutaneous Transluminal Coronary Artery (PTCA), an occluded artery is revascularized by directing a guidewire through the occluded artery to a location just beyond the occlusion. A balloon catheter is then conveyed along the guidewire to the occlusion, where the balloon is then inflated to predetermined pressures at predetermined intervals to revascularize the artery. However, in the case of a total occlusion, PTA or PTCA cannot be performed because there is no channel within the occlusion through which the guidewire may be inserted.

In addition, there are other angioplasty systems, some of which are described below, in which it is either necessary or advantageous to be able to insert a guidewire through an occlusion in an artery in order to ablate the occlusion with laser energy.

For the above and other applications the guidewire system of the present invention was developed.

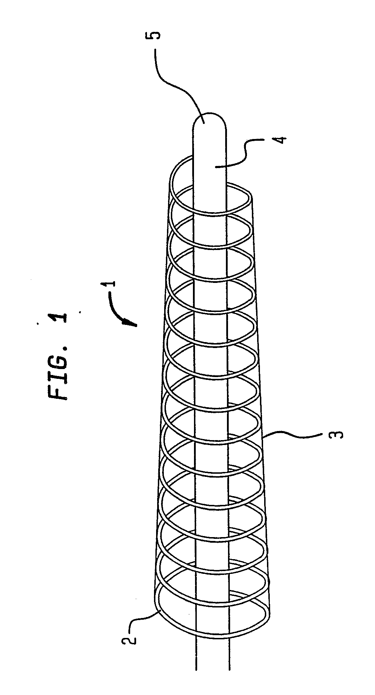

With reference to Figure 1, the guidewire 1 of the present invention includes a flexible wire that is wound into a helical coil 2 having an outer diameter of about 0.015 inches. The wire is preferably made of stainless steel with a diameter of about 0.0015 inches. At the distal end of the coil 2, the diameter of the coil 2 is reduced gradually to form a tapered portion. The coil 2 is coated with a plastic coating 3. Preferably the coating 3 is about 0.0015 inches thick and is made from a material having a low coefficient of friction, such as teflon, so as to provide a smooth, low friction surface.

Mounted within the hollow lumen of the coil 2 is a flexible silica fiber 5, that has a diameter of about 200 microns. The distal end of the silica fiber 5 is bonded to the distal end of the guidewire 1 by epoxy or other known means.

In addition, a safety wire (not shown) may be soldered to the distal tip of the wire 2. The safety wire preferably has a diameter between 0.006 and 0.008 inches, and is made from stainless steel with a rounded tip coated with a lubricious coating. The tip may be provided with an optical opening.

The silica fiber 5 provides two functions within the guidewire. First, it acts as a reinforcement for the guidewire coil 2. Second, it delivers pulsed laser energy to an occlusion that is completely blocking an artery so that an opening may be ablated through the occlusion in order to enable the guidewire to pass through the occlusion.

Although different kinds of laser energy may be used with the guidewire of the present invention, it is preferable to use a high energy, pulsed, ultraviolet laser, such as an Excimer laser.

In using the guidewire of the present invention in a PTA or PTCA procedure, the guidewire 1 is inserted into the occluded artery and directed through the artery to the occlusion in a manner known to those skilled in the art. With the distal end of the guidewire 1 adjacent the occlusion, laser energy is then transmitted through the silica fiber 5 to ablate a small opening in the occlusion. Once the opening has been ablated, the guidewire 1 is then able to pass through the occlusion. With the guidewire 1 inserted through the occlusion, a conventional balloon catheter is then conveyed along the guidewire to the occlusion, where the balloon catheter is then inflated to predetermined pressures at predetermined intervals in order to revascularize the artery according to conventional PTA or PTCA techniques.

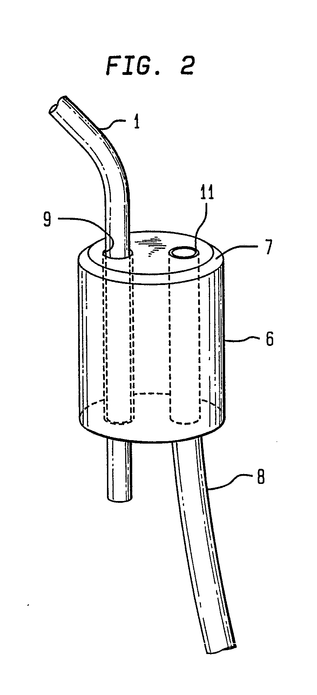

As indicated above, the guidewire 1 of the present invention may also be used with a variety of fiber optic instruments used for angioplasty. Referring to Figure 2, a preferred embodiment includes the use of the guidewire 1 with a sleeve 10 and a fiber optic instrument 12.

The sleeve 6 is preferably between 1 and 200 centimeters in length, and has a rounded tip 7 at its distal end. The tip 7 can be made of stainless steel and glued or welded to the sleeve, or it can be formed integrally on the sleeve 6. The diameter of the tip can vary from 1.2 to 2.5 mm, depending upon the size of the blood vessel. The rounded tip 7 serves as both a dilator to enlarge the blood vessel and as a device to blunt the tip of the optical fiber instrument 8 so as to minimize trauma to the blood vessel.

The sleeve 6 has at least two lumens 9, 11 therein. A first lumen 9 is designed to accept the guidewire 1 and is preferably within the range of twelve-thousandths (.012) to thirty-eight thousandths (.038) of an inch in diameter. The diameter of the first lumen may vary, depending on the diameter of the guidewire 1.

The second sleeve lumen 11 is designed to enclose the optical fiber instrument 8, or an array of fibers, if such is the case. The diameter of the second lumen 11 may also vary according to the diameter of the optical fiber instrument 8 or fibers being used. The distal end of the optical fiber instrument 8 is bonded within the second lumen 11 by any suitable means well known to those skilled in the art of bonding.

To use the guidance system, the guidewire 1 is threaded through the lumen of the blood vessel by means of an introducer catheter (not shown). The guidewire 1 is inserted up to the location of a total obstruction in the vessel, or in the case of a subtotal lesion, beyond the lesion.

In the event of a total obstruction, laser energy is delivered through the silica fiber 5 in the guidewire to ablate a small opening in the obstruction through which the guidewire may pass.

The sleeve 6 is then mounted onto the guidewire 1, with the guidewire 1 extending through the first lumen 9 of the sleeve 6. The sleeve 6 and the optical fiber instrument 8, which is bonded thereto, are then advanced along the guidewire 1 until the sleeve 6 and the distal tip of the optical fiber instrument 8 are adjacent the lesion to be ablated. The combination of the guidewire 1 and the sleeve 6 ensures that the optical fiber instrument 8 remains in alignment with the blood vessel, thus avoiding perforation of the blood vessel by the tip of the optical fiber instrument 8 during positioning of the fiber or by the laser beam during ablation.

Alternatively, the guidewire 1 may initially be threaded through the first lumen 9 of the sleeve 6, prior to the insertion of the guidewire 1 into the blood vessel.

In a preferred embodiment, the second lumen 11 is eccentrically located within the sleeve 6. In such an arrangement, rotation of the optical fiber instrument 8 while it is in the blood vessel causes rotation of the sleeve 6 which causes the radial position of the optical fiber instrument 8 to shift within the blood vessel. Accordingly, rotating the optical fiber instrument 8 during lasing causes a larger lumen to be ablated within the blood vessel.

Once the lasing is completed, the sleeve 6 and the optical fiber instrument 8 can be withdrawn, leaving the guidewire 1 in place within the blood vessel. Angiographic dye can then be injected through a guiding catheter around the guidewire 1 to evaluate the results of the lasing operation. If the results are unsatisfactory, the entire procedure can be repeated, possibly using different laser parameters or fibers.

For a more complete understanding of the present invention, details of other various fiber optic instruments that can be used with the guidewire 1 of the present invention will now be described.

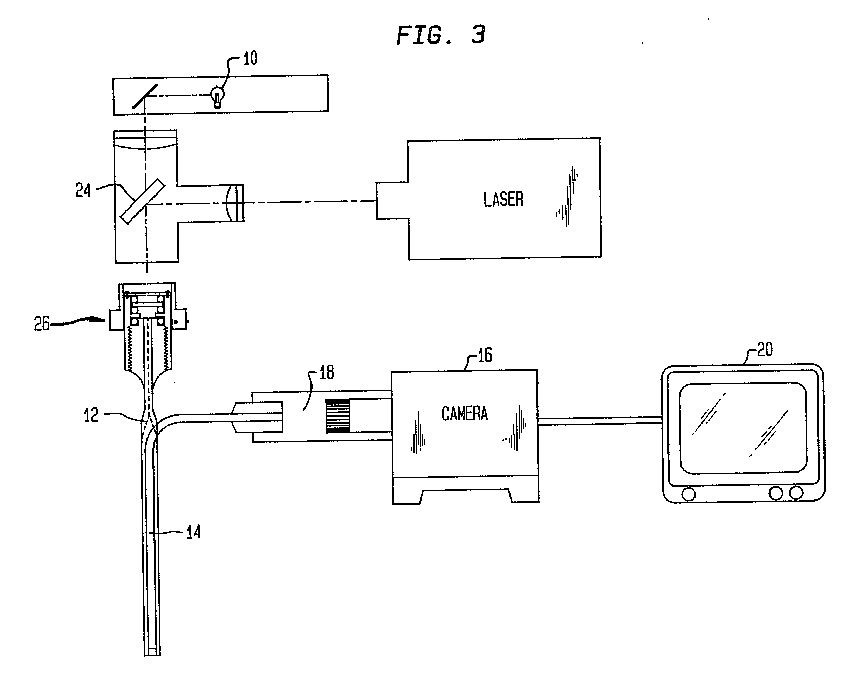

In the following description, a laser delivery system is described with particular reference to the use of Excimer laser energy in an angioplasty system. Referring to Figure 3, an angioplasty arrangement is shown in schematic form. The angioplasty system should be capable of performing three functions within the blood vessel. The first two of these relate to the illumination and imaging of the interior of the vessel to enable a physician to successfully propagate the distal end of the system through the vessel to the location of the obstruction. Accordingly, the output from a source of visible light, such as a Halogen or Xenon lamp 10, is directed to the proximal end of an optical fiber 12. The distal end of this fiber is housed within a catheter (not shown) to enable it to be fed through a blood vessel. A second optical fiber 14 located adjacent the fiber 12 within the catheter receives the image from the illuminated interior of the blood vessel and transmits it to a video camera 16 by means of a video coupler 18 connected between the output end of the fiber 14 and the camera. The image presented to the camera 16 by the fiber 14 is converted into a video signal and fed to a suitable monitor 20 for viewing by the physician as the catheter is being positioned inside the blood vessel. Alternatively, the video coupler, camera and monitor can be replaced by an eyepiece that is attached to the proximal end of the fiber 14.

Once the distal ends of the fibers 12 and 14 have been appropriately positioned adjacent the obstruction, a high energy pulsed ultraviolet laser, such as an Excimer laser, is activated to ablate the obstruction. In a preferred implementation of the invention, the laser light is conducted along the same optical fiber 12 as the visible light. To accomplish such a result, the output beam of the laser is directed at a beam splitter 24 which also transmits the visible light from the source 10. These two forms of light energy are propagated along the same path and presented to the input end of the optical fiber 12 by means of an energy coupler 26.

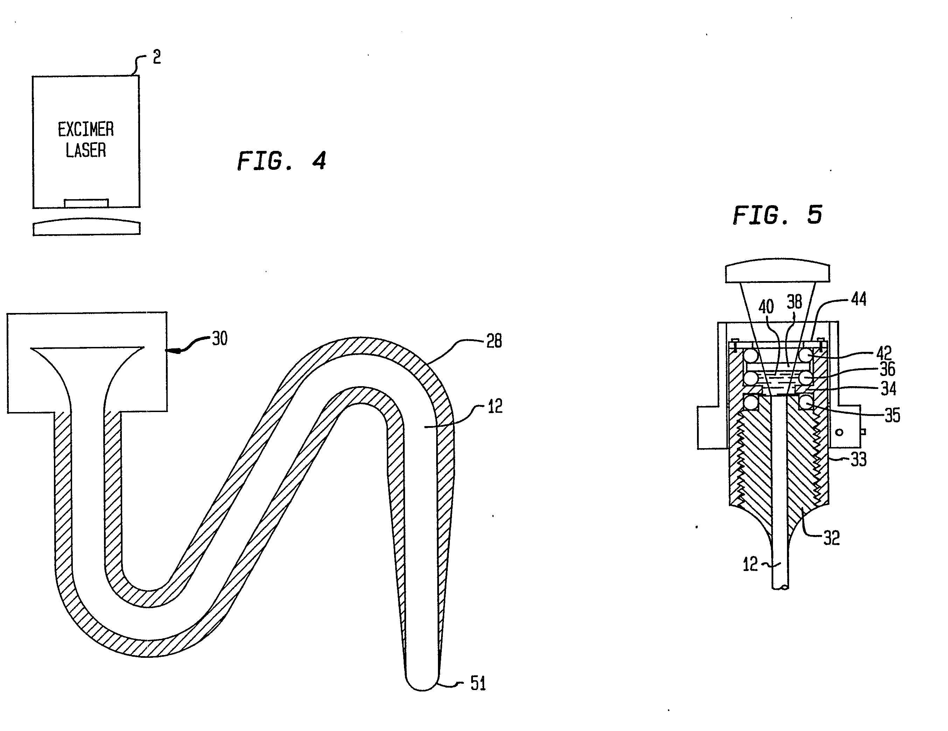

Referring now to Figure 4, one embodiment of the delivery system for high energy pulsed laser light is illustrated in greater detail. The delivery system comprises two basic elements. One of these is the optical fiber 12, and the other is the energy coupler. A fiber that is particularly suitable for use in the delivery of high energy pulsed ultraviolet laser light is a multi-mode fiber which has a relatively large core, or active area, relative to the area of its cladding, i.e., the outer skin of the fiber. The core is made of substantially pure synthetic fused silica, i.e., amorphous silicon dioxide. This material preferably has a metallic impurity content of no more than 30 parts per million, to provide better conduction of the transmitted laser energy than that which is obtainable with natural fused quartz. The term "metallic impurity" includes both metals per se and oxides thereof.

Even with such a low level of metallic impurity, defects in the silica fiber can serve as linear and non-linear absorption sites for the photons. These defects can vary from oxygen vacancy to unbonded silicon atoms found in any silica glass. They can result in lowered transmittance of ultraviolet radiation. Increasing the intensity (or energy) of the laser light that is introduced into one end of a fiber exhibiting such defects will not necessarily result in proportionally increased output at the other end. Rather, the increased intensity level can reduce the threshold level at which bulk damage occurs to the silica glass, and thereby destroys the delivery system.

The transmittance of high energy UV laser light in a fiber made of synthetic silica is enhanced by lightly doping the silica with a material which functions to repair some of the inherent structural defects of the silica. The silica is preferably doped with an OH⁻ radical, to thereby form so-called "wet" silica. It is believed that defects in silica that affect UV light transmission comprise oxygen hole centers and unbonded silica atoms. It is theorized that the doping of the silica with the OH⁻ radical functions to repair these defects by eliminating the oxygen holes or vacancies in one case and by bonding to the silicon to form the $SiO_2$ double bond. It has been reported that pure silica having only about 5 parts per million (ppM) of an OH radical has an absorption coefficient which is 2-3 times greater than silica having about 1200 ppM of the radical. See J.H. Stathes et al, Physical Review B., Vol. 29, 12, 1984, pp. 70-79. Other investigations have reported that an optical absorption band appears in silica fibers having a low OH⁻ content as a result of the fiber drawing process. See Kaiser et al, J. Opt. Soc. Am. 63, 1973, p. 1141 and J. Opt. Soc. Am. 63, 1974, p. 1765. Apparently, an increase in the OH⁻ content of silica reduces both types of absorption sites described above, and in accordance with the present invention this concept is applied to a system for delivering high peak energy ultraviolet laser pulses to thereby enhance the efficiency of the energy transmittance. Preferably, the silica that makes up the fibers contains about 200 to 2000 ppM of the OH⁻ radical, most preferably 1200 ppM.

In another embodiment of the optical fiber instrument, the silica that is used to produce the fibers of the delivery system is doped with fluorine. Fluorine doped silica exhibits even lower attenuation than high OH silica. It appears that the fluorine functions to shift the absorption band gap in the $SiO_2$ structure, to facilitate the transmittance of a large number of photons at low wavelengths. For multimode fibers having diameters in the range of 100 micrometers to 1500 micrometers, the silica preferably should contain between 0.25 and 2.0 wt% fluorine, most preferably 1.0 wt%.

The silica can be doped with both the OH⁻ radical and fluorine. When both of these materials are used in combination, the OH radical content should range between 200 and 2000 ppM, and the fluorine should comprise between 0.5 and 3 wt% of the silica.

In the context of the present invention, the fiber can be a single fiber or a bundle of fibers having a total diameter in the range of 100-2,000 microns. A bundle of close-packed small-diameter fibers is preferred because they provide greater overall flexibility and thereby more easily accommodate the twists and tight turns that are required to feed the delivery system through body cavities. This is particularly desirable where a larger diameter waveguide is required to deliver a relatively large diameter beam with uniform intensity, such as in vascular angioplasty. This entire structure can be surrounded by a protective flexible jacket 28 made of a material which is not damaged by ultraviolet light. More particularly, when the fiber undergoes sharp bends, for example at the juncture of two arteries, light losses occur. These losses may be enough to melt some types of jacket materials such as Silicone and nylon. However, UV light resistant materials, for example UV cured acrylate compound or Teflon , can sustain high bending losses without degradation and are therefore more desirable for the jacket.

In one embodiment, the protective jacket is incorporated as part of the fiber itself, rather than being a separate piece of structure which surrounds all of the fibers. As noted previously, every fiber comprises a core and a cladding which surrounds the core to maintain the transmitted light energy within the core. The cross-sectional area of the fiber might normally have a core/cladding ratio of 80/20 to provide suitable flexibility. Typically, both the core and the cladding are made of glass, with the cladding being appropriately modified (e.g., doped) to provide it with a lower index of refraction. In this conventional structure, the protective jacket comprises a third layer which surrounds the core and cladding.

In accordance with one aspect of the invention, the conventional glass cladding is eliminated and the core of the fiber is directly surrounded by a coating of organic material. One specific preferred material is UV-cured acrylate. It has a lower index of refraction than silica, and thereby functions to maintain the laser energy within the core. It also serves to protect the silica glass, and hence eliminates the need for a third layer. This reduces the overall size of the fiber and hence enables the net cross-sectional area of the core to be increased for a delivery system having a given outer diameter.

Further details regarding the composition of preferred coatings can be found in U.S. Patent No. 4,511,209, the disclosure of which is incorporated herein by reference.

A silica fiber of this construction can typically accommodate input energy up to a level around 30 mJ/mm² produced by a commercially available Excimer laser with a 10-40 pulse. If the density of the energy is increased above this level, the input end of a conventional fiber having a planar, polished surface will be damaged or destroyed if the laser is applied directly to it. Unfortunately, this density level is about the minimum that is required to produce ablation of calcified plaque, thus providing no tolerance range if the intended use of the delivery system is for angioplasty. Accordingly, to enable a higher level of energy to be conducted in the fiber, an energy coupler 38 can be provided at the input end of the fiber. In the embodiment illustrated in Figure 4, this energy coupler comprises a section of fiber that has a larger cross-sectional area than the main portion of the fiber. This larger cross-sectional area gradually tapers to the nominal diameter of the fiber, to provide a funnel-shaped input section.

Production of such a shape on the end of the fiber can be accomplished by appropriate design of the die through which the silica is drawn to produce the fiber. By interrupting the drawing of the fiber, a bulbous mass remains at one end of the fiber. This mass can be cut and polished to produce the funnel-shaped input section.

In operation, the increased area of the funnel-shaped coupler decreases the input energy density for a given level of energy within the fiber. Accordingly, the area of the input end can be appropriately dimensioned to enable a sufficient amount of energy for ablation of tissue to be coupled into the fiber without damaging the input end. Once it has been coupled in, the density of the energy is increased by decreasing the cross-sectional area of the fiber within the tapered section, so that a greater amount of energy can be conducted within the fiber than would be possible without such a device.

Another embodiment of an energy coupler is illustrated in Figure 5. In this embodiment, the optical fiber has a uniform diameter along its length and terminates at a flat polished end. The end section of the fiber is encased within a ferrule 32 made of a suitable material such as brass, for example. An aluminum casing 33 having an annular ring 34 projecting from the inner wall thereof is threaded onto the ferrule. A TEFLON O-ring 35 disposed between the end of the annular ring and the ferrule provides a watertight seal between the casing and the ferrule. A second O-ring 36 is disposed on top of the annular ring and supports a glass plate 38 made of z-cut quartz, for example. This arrangement forms a fluid-tight cavity 40 between the ferrule 32, the casing 33 and the glass plate 38. The glass plate can be held in place by means of a third O-ring 42 and a clamping ring 44 disposed on the top of the casing. The fluid tight cavity is filled with liquid which acts as a buffer to the input end of the fiber, enabling laser energy having a relatively high density to be coupled into the fiber without damage thereto. The liquid within the cavity can be distilled and deionized water, or it can be a transparent oil having an index of refraction that is matched to that of the fiber 12, for example.

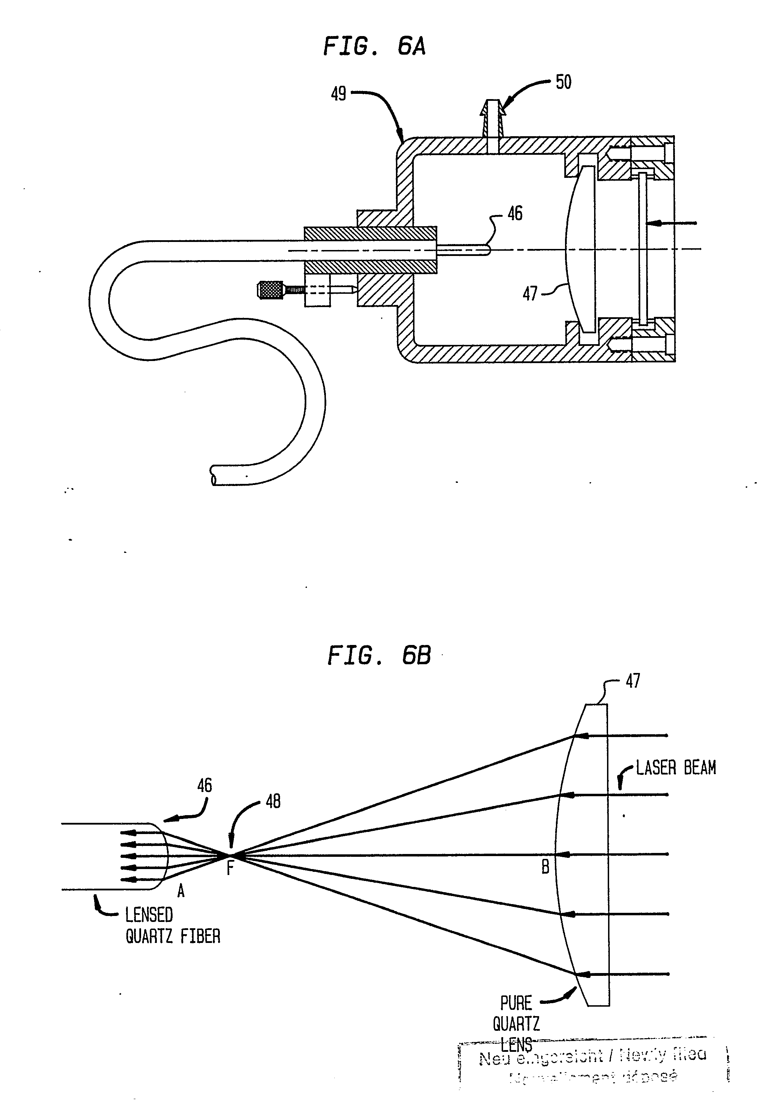

Another embodiment of an energy coupler is illustrated in Figures 6A and 6B. In this embodiment, the input end of the fiber is provided with a fused semispherical lens 46. This lens can be formed by melting the material of the fiber itself with a microtorch, to produce a high purity silica lens with no impurities or cracks. Alternatively, the lens 46 can be a separately ground lens that is attached to the flat end of the fiber. The fiber 12 can be tapered as shown in Figure 4, or it can have a uniform diameter along its length.

A second lens, preferably a plano-convex lens 47, focuses the input beam from the laser to a focal point 48. The input lens 46 on the fiber is axially aligned with the lens 47 and is located at a distance from the lens 47 which is greater than the focal length of that lens. Thus, the focused laser energy appears to be coming from a point source. The lens 46 collimates this focused energy and couples it into the fiber.

The input end of the fiber with the lens 46 and the focusing lens 47 are housed within a chamber 49. This chamber is provided with a vacuum port 50 to enable the chamber to be evacuated of air. If air were present between the lenses 46 and 47, the highly concentrated energy at the focal point 48 might cause a breakdown of nitrogen and oxygen gases that could contaminate the lens 46. In addition, the vacuum environment keeps out dust and other particles which could settle on the lens 46 and act as a heat sink, destroying the roundness of the lens.

Alternatively, this chamber 49 can be filled with a liquid, such as water or oil for example, which matches the index of refraction of the silica fiber. The higher index of refraction of the liquid reduces the dielectric shock when the pulse propagates from the liquid transmission medium to the fiber, relative to that which is experienced when air is the transmission medium.

Although the preferred embodiment employs a curved lens at the proximal input end of the fiber, it is possible to couple the energy into a fiber having a planar input surface. However, it is important to ensure that this surface is free of scratches and other imperfections. This can be accomplished by heating the end of the fiber with a micro-torch to cause the fiber material to melt and flow slightly, thereby removing the imperfections caused by polishing.

The type of energy coupler shown in Figure 6A serves to amplify the energy within the fiber. More particularly, the amplification factor is equal to the ratio of the diameter of the laser beam at the lens 47 to the diameter of the fiber. This ratio is also related to the magnification produced by the two lenses. Referring to Figure 6B, the dimension FB is the focal length of the lens 47 and the dimension FA is the distance between the lens 47 and the focal point 48. The magnification $\frac{FB}{FA}$ factor of these two lenses is defined as . Since this factor must be equal to the laser energy amplification, the appropriate distance between the lenses 46 and 47, i.e., AB = FB + FA, can be determined from the following relationship:

$$\frac{FB}{FA} = \frac{D_L}{D_F}$$

where $D_L$ is the diameter of the laser beam and $D_F$ is the diameter of the fiber.

Although illustrated as a separate element in the figures, it will be appreciated that the energy couplers could be incorporated into the structure of a laser, to provide an integrated laser and coupling system.

Thus, with the combination of the lightly doped synthetic silica fiber and the energy coupler 30 that enables a greater level of energy to be conducted through the fiber, an amount of high energy laser light that is sufficient to produce an incision can be safely transmitted through an optical fiber waveguide without the risk of damage to the fiber.

To further increase the peak energy that is delivered through the system it is preferable to slightly increase the length of the pulses beyond the relatively short duration that is typically produced by commercial Excimer lasers and the like. For example, a pulse having a duration in the range of 30-1000 nsec, more preferably 100-300 nsec, enables much higher peak energy to be applied with the same delivery system than a 10 nsec pulse yet is still sufficiently short to produce the desired cutting action. One example of a circuit for stretching the output pulses of a laser is the magnetic switch developed at the Jet Propulsion Laboratory by Drs. J. Ladunslager and T. Tacala. In this regard, it is not necessary that each lengthened or stretched pulse comprise a single, continuous pulse having a duration of 100-300 nsec, for example. Rather, it could comprise a burst of shorter length successive pulses which together provide an effective pulse length of the desired duration.

With the increased energy that is provided by the lengthened pulses, the energy level within the fiber will likely be more than sufficient to enable the laser beam to ablate an obstruction. In fact, the beam could be expanded as it exits the fiber and still contain sufficient energy density to ablate tissue. By expanding the diameter of the laser beam, for example by means of an increasing taper or fusing a larger diameter fiber at the distal end of the fiber, a larger area of tissue is ablated to produce more favorable results towards obtaining better blood flow in a blood vessel while using a small diameter flexible fiber that can be easily propagated through the vessel.

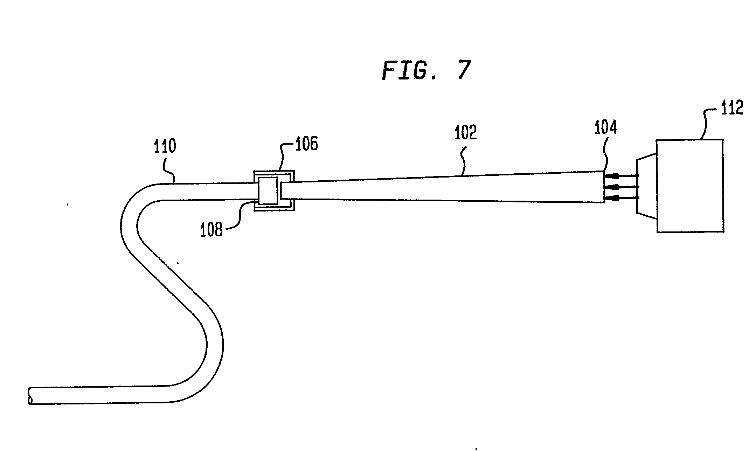

In an additional method of coupling a substantially pure silica fiber delivery system to the high energy pulsed laser, an energy coupler may be used. With reference to Figure 7, a pure silica fiber 102 is tapered at about a one degree (1 ) taper and is about 30 centimeters long. The fiber 102 has an input end 104 with a diameter of about 15mm. At the narrow end 106 of the fiber 102 is a modular connector 106 that connects with a connector 108 on the end of the optical waveguide 110.

A source 112 of laser energy propagates laser energy into the tapered fiber 102 such that, in a preferred embodiment the energy density at the connector 106 is about 50-100 milliJoules/mm$^2$.

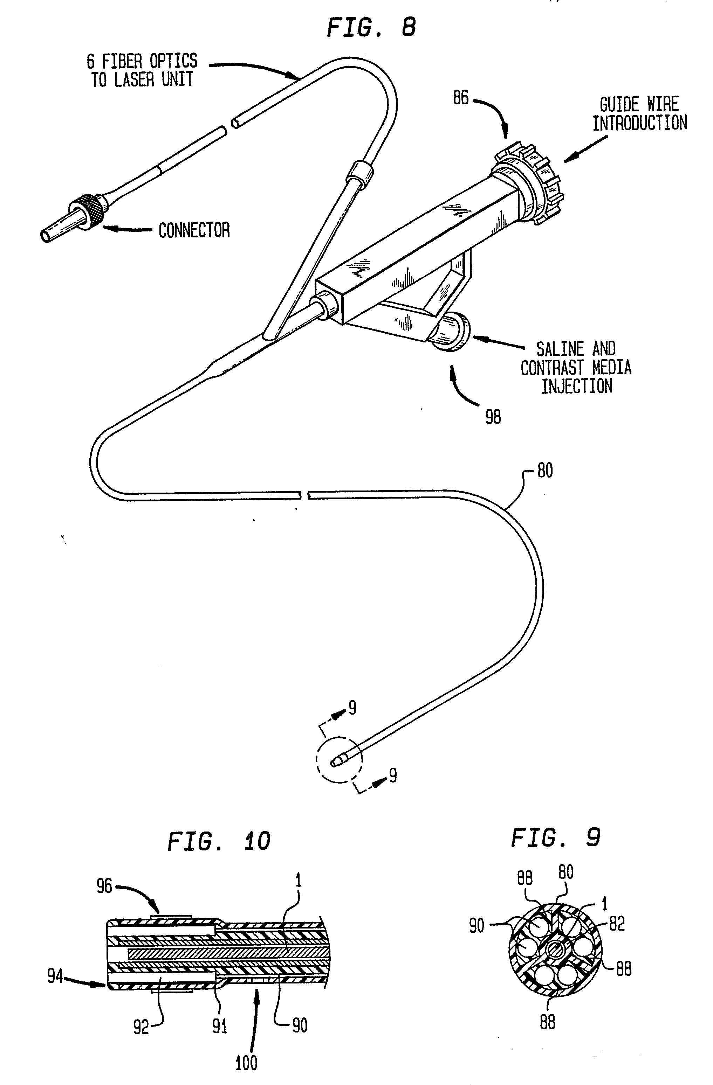

A further embodiment of a catheter for coronary laser angioplasty, which operates in accordance with the foregoing principles, is illustrated in Figures 8-10. The catheter 80 is multi-compartmented. It includes a center lumen 82 which accommodates the guidewire 1. The guidewire 1 is introduced through a suitable coupling device 86 at the proximal end of the catheter. The center lumen 82 is surrounded by a plurality of circumferentially disposed outer lumens 88. These lumens 88 each house one or more substantially pure synthetic silica fibers 90. In the illustrated embodiment there are three outer lumens 88 each housing two fibers 90. It will be appreciated that a different number of lumens and/or fibers per lumen can be employed, as determined by the relative sizes of the fibers 90, the guidewire 1 and the diameter of the catheter. Furthermore, the guidewire lumen may be eccentrically arranged within the catheter.

An enlarged cross-sectional side view of the distal end of the catheter is shown in Figure 10. Here, each laser-energy conducting fiber 90 is fused at its end face 91 to a short section of a larger diameter fiber 92. For example, the fibers 90 might have a diameter of 200 microns throughout the length of the catheter 80, and the short end fibers 92 might have a diameter of 300 microns, or a 100 micron diameter fiber may have a short 200 micron diameter fiber fused to its end. Each end fiber 92 can be about 3mm long, and can be made from the same silica material as the fibers 90. By virtue of the larger diameter fiber at the distal end, the laser beam can expand as it emerges from the fiber, thereby providing a larger area of coverage and subsequently a larger ablation area. Furthermore, the plural fibers located symmetrically around the guidewire provide uniform energy distribution over a larger area.

The fibers 92 are held in place at the end of the catheter by means of a suitable epoxy 94. A gold marker ring 96 can be provided around the catheter at the distal end, to assist in locating the end of the fiber during a fluoroscopy and angiography procedure.

Except at the very end of the catheter where the epoxy 94 is present, there is free space within each outer lumen 88 between the fiber 90 and the walls of the lumen. If desired, this free space can be used to provide a saline solution, or other contrast media, to the site of the obstruction. The solution can be injected into the catheter through a suitable port 98 at the proximal end, and emerge through holes 100 in the side wall of the catheter at its distal end (see Figure 10).

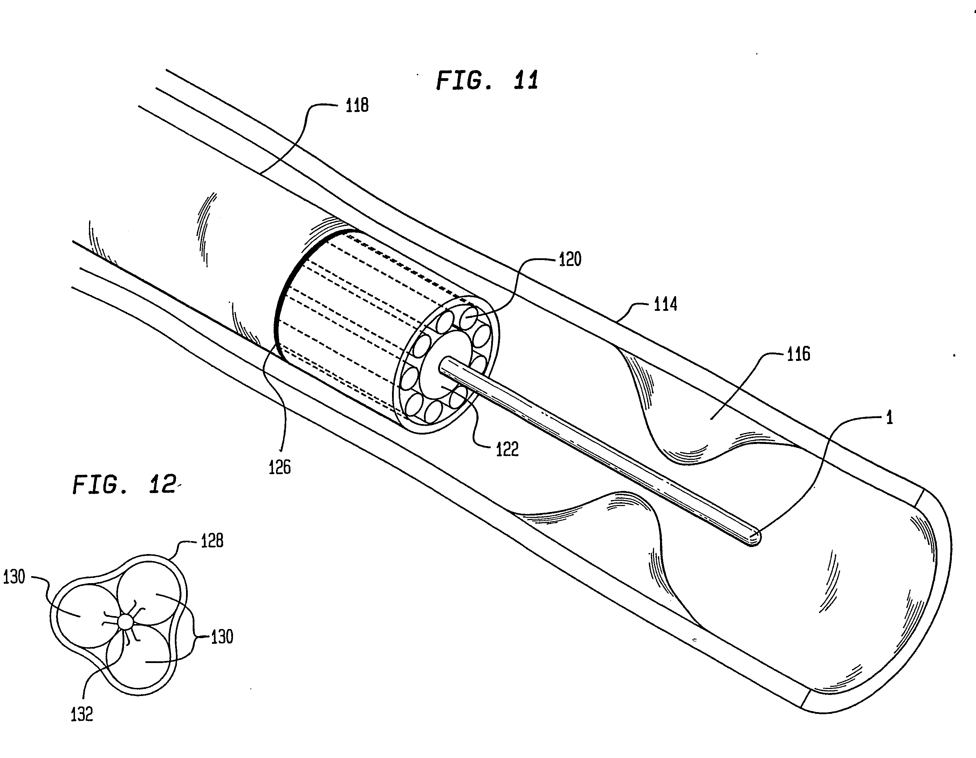

In certain situations, there is a need to ablate a large area of a fully occluded vessel in order to maintain an open lumen. The clinical efficacy of ablation in the large peripheral vessels is enhanced when the lumen created is larger than 5mm. Long term reocclusion of large vessels with low pressure blood flow is reduced when the energy delivery system is used to ablate a hole without subsequent dilation of the vessel. The following can also be used with the guidewire 1 of the present invention to make large diameter holes in blood vessels using a small overall diameter delivery system. Turning attention now to Figure 11, a blood vessel 114 is shown with a lesion 116 therein. A catheter 118 includes a plurality of optical fibers 120 evenly distributed about a concentrically mounted inflatable balloon 122. The optical fibers 120 may be 300-400 microns. The guidewire 1 is concentrically located in a center lumen within the balloon 122. If desired, a metallic (gold) marker 126 may be located adjacent the distal end of the catheter, such that the catheter 118 may be located by an x-ray or fluoroscopy system.

In operation, the guidewire 1 is inserted through the lumen or blood vessel 114 until the guidewire 1 is adjacent or within the lesion 116 that is to be ablated. The catheter 118 is then conveyed along the guidewire 1 until the distal end of the catheter 118 is also adjacent the lesion 116. The fibers 120 deliver an initial dose of high pulse laser energy to ablate the inner portion of the lesion 116. Subsequently, the balloon 122 is inflated to a predetermined pressure which then forces the fibers 120 into an array of a larger diameter. A subsequent delivery of high pulsed laser energy is then delivered to ablate the outer peripheral portions of the lesion 116. The balloon 122 may be additionally inflated if necessary to obtain an array of fibers 120 of a still larger diameter.

With reference to Figure 12, an additional embodiment is disclosed. A catheter 128 has three balloons 130 located in an equally spaced arrangement at the distal end of the catheter 128. A first lumen 132 is positioned concentrically among the balloons 130. In the first lumen 132 is disposed a fiber optic instrument 133. If desired, the guidewire 1 may be retained in a second lumen adjacent the first lumen 132. Additional lumens may also be included at the center of the balloons 130 to accommodate other instruments to assist with illumination or flushing.

In operation, the lumens at the center of the catheter may be positioned or tilted by selectively inflating and deflating the three balloons 130. In one mode of operation, the balloons may be inflated sequentially and continuously so as to selectively revolve the fibers in a circular pattern along the perimeter of the catheter. The fibers may be centered by inflating all of the balloons.

In another embodiment, two, four or any other number of balloons may be used instead of three.

As in the embodiment shown in Figure 11, the embodiment shown in Figure 12 may also include a marker, such as a gold band, at the distal end of the catheter.

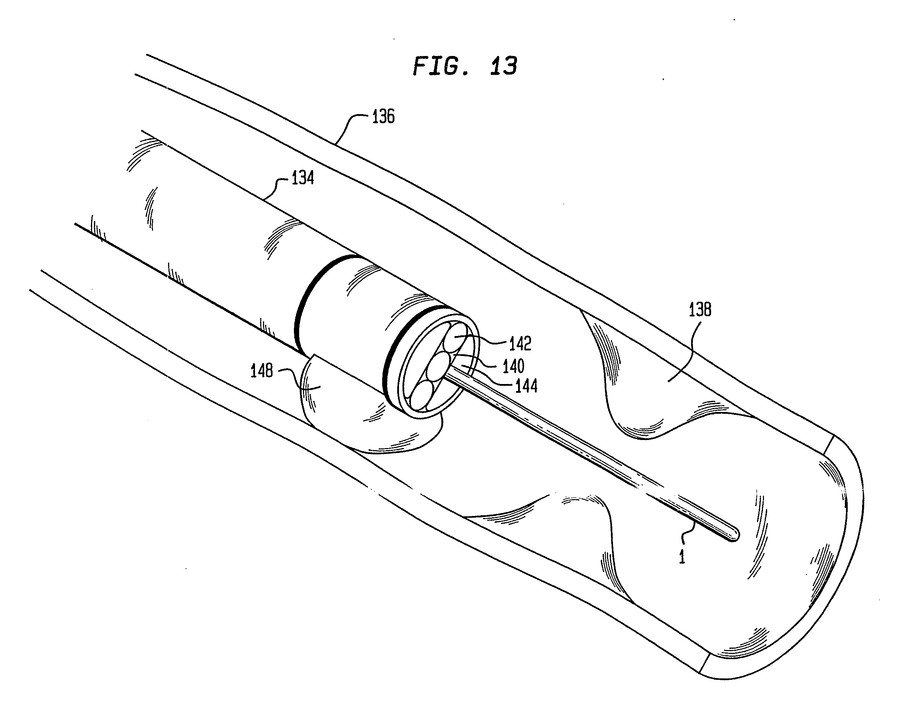

Figure 13 discloses an additional preferred embodiment of a catheter 134. A center lumen 140 extends diametrically from one edge of the catheter to an opposing edge and contains an array of fibers 142. The guidewire 1 is provided in a guidewire lumen 144 that is provided at one edge of the catheter 134. A balloon 148 is mounted outside of the catheter 134 at one side thereof. The balloon 148 can be made of a high pressure balloon material commonly used in Percutaneous Transluminal Angioplasty (PTA) and Percutaneous Transluminal Coronary Angioplasty (PTCA) in order to maintain an accurate, repeatable size upon inflation.

The guidewire 1 is inserted through a blood vessel 136 until it is adjacent a lesion 138 in the blood vessel. The catheter 134 is then conveyed along the guidewire 1 until the catheter 134 is adjacent the lesion 138. In operation, the balloon 148 is inflated so as to contact the wall of the blood vessel 136. Ablation is then carried out while rotating the catheter 134 about the guidewire 1 in order for the fibers 142 to revolve through a complete revolution. The process can be repeated by further inflating the balloon 148 to a larger diameter and again rotating the catheter 134 while ablating the lesion with laser energy.

One or more gold bands 147 may be provided at the distal end of the catheter 134 so as to enable detection of the catheter by fluoroscopy or x-ray.

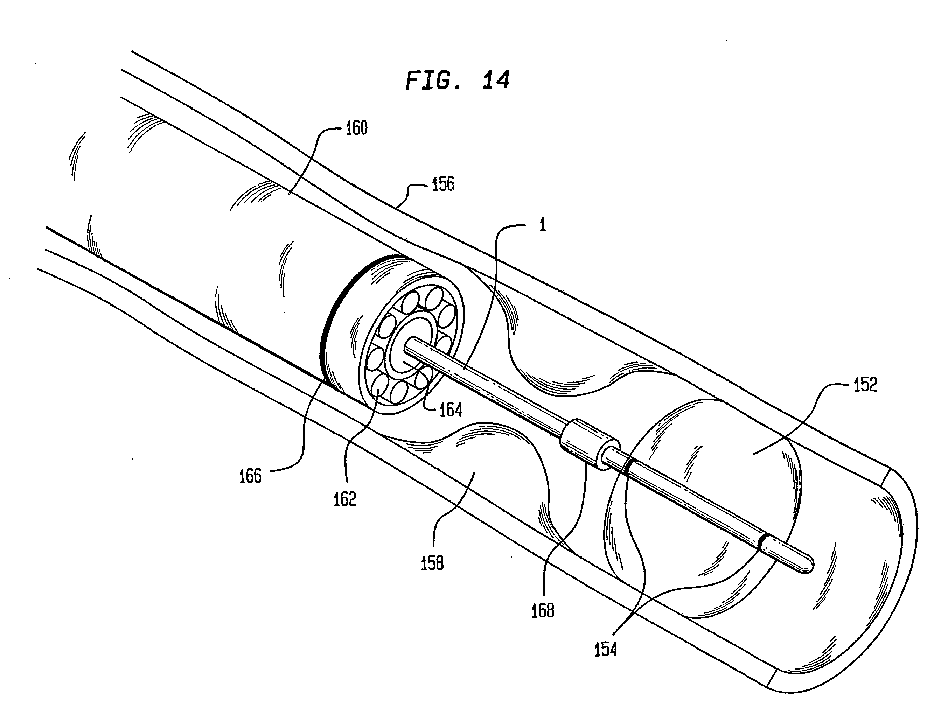

Another preferred embodiment of the present invention is illustrated in Figure 14. In this embodiment the guidewire 1 has a balloon 152 at the end thereof. Markers 154 are mounted on the guidewire 1 adjacent the balloon 152. The guidewire 1 is disposed through a guidewire lumen 164 in the center of a catheter 160. The catheter 160 further includes a plurality of fibers 162 arranged in an annular pattern about the guidewire lumen 164. If desired, a marker 166, such as a gold band, can be placed around the distal end of the catheter 160 to facilitate detection of the catheter.

In operation, the guidewire 1 with the balloon 152 at one end thereof is inserted through the blood vessel until it is located beyond the lesion 158. The balloon 152 is then inflated, thus centering the guidewire 1 within the vessel. Such centering of the guidewire 1 minimizes the likelihood that the catheter 160 will contact the walls of the blood vessel 156 during the ablation process. A stop 168 may be placed on the guidewire 1 so as to prevent the catheter 160 from contacting and thus possibly rupturing the balloon 152.

The fibers 162 in the catheter preferably range from about 50 to 300 microns. The guidewire lumen 164 is large enough to allow free movement over a guidewire ranging from 0.014 to 0.038 inches in diameter.

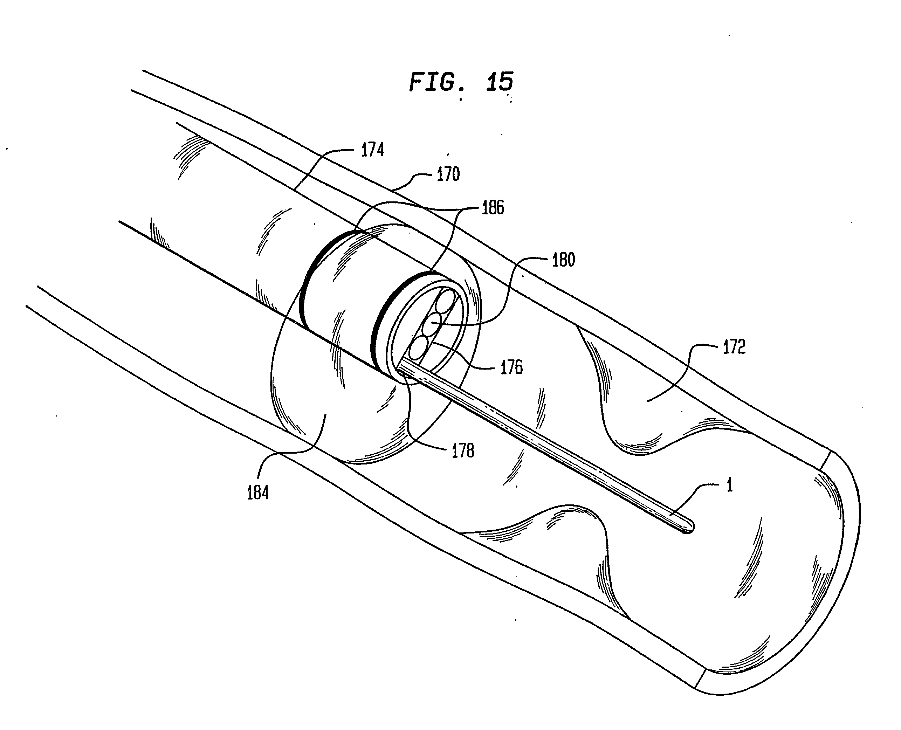

With reference to Figure 15, another embodiment is disclosed within a blood vessel 170 having a lesion 172 therein. A catheter 174 includes a guidewire and flushing lumen 178 located adjacent an inner edge of the catheter. An optical fiber lumen 176 has a width that extends from the guidewire and flushing lumen 178, through the center of the catheter to the edge of the catheter diametrically opposite the guidewire and flushing lumen 178. A plurality of optical fibers 180 made of substantially pure silica are disposed within the optical fiber lumen 176, and the guidewire 1 is disposed within the guidewire and flushing lumen 178. A balloon 184 is bonded at one point to the outer surface of the catheter 174.

To operate, an initial ablation is performed with the balloon 184 in an uninflated condition. The ablation is effected by delivering a high energy pulsed laser, such as from an Excimer laser, through the optical fibers 180 while rotating the fibers about the guidewire 1. The rotation enables the fibers to ablate a circular area about the size of the catheter outer diameter.

In the next stage, the outer balloon is inflated, thus urging the fibers into a larger diameter. A second ablation is performed with the balloon inflated and while rotating the catheter so as to ablate an annular area having a diameter larger than the diameter of the circular area ablated during the first stage. During the second stage, the balloon is inflated to a predetermined size in order to ensure that the second annular area is contiguous to the circle ablated during the first stage.

The Excimer energy is only capable of ablating a lesion in a forward direction and does not require that the blood between the lesion and the optical fibers be displaced with a solution, such as saline, that is transparent to the laser. In addition, the laser transmitted through the optical fibers is capable of ablating a lesion in a blood field. Therefore, there is no necessity to block the blood flow around the catheter during the ablation process.

The tip of the catheter should have a round, conical, distal tip to minimize the trauma to the vessel wall. The optical fibers 180 comprise a highly flexible array of fibers that may range in size from 100 to 400 microns in diameter, with a possible enlarged fiber tip output diameter to create a large ablation area. The fibers which are immobilized at the distal end of the catheter by epoxy are polished to form the round distal tip.

If desired, markers 186, such as gold bands can be placed at the distal of the catheter to facilitate monitoring of the catheter by x-ray or fluoroscopy.

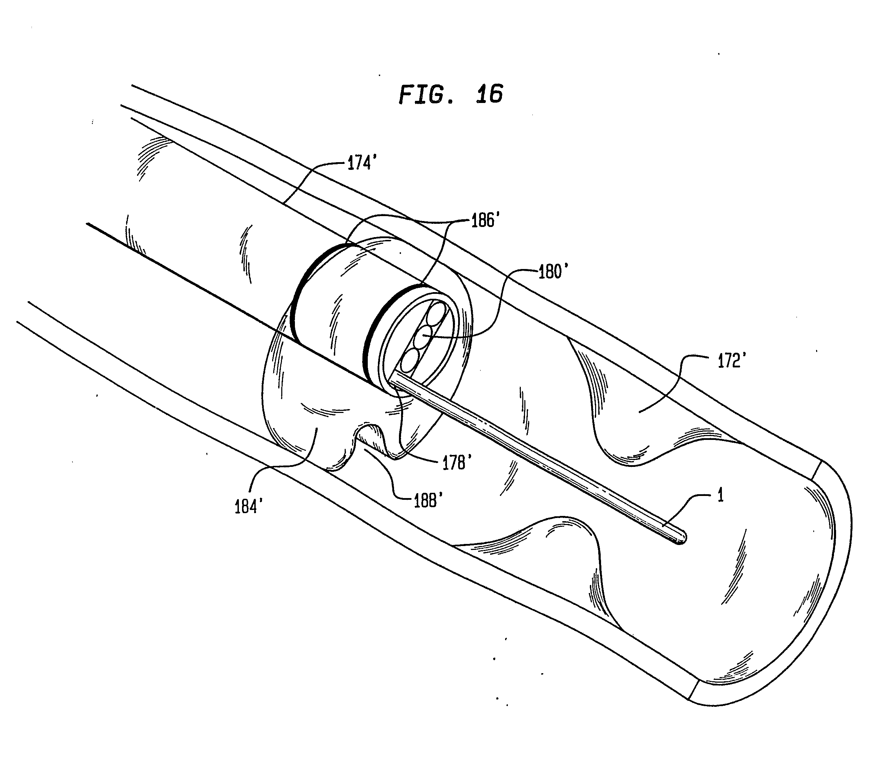

With reference to Figure 16, a catheter 174' is disclosed that is substantially the same as the catheter 174 of Figure 15, described above. The significant difference between the catheter 174 of Figure 15 and the catheter 174' of Figure 16 is that the balloon 184' of Figure 16 includes a recess 188' that enables blood to flow around the balloon 184' during its use.

The off-center balloon catheters of Figures 13, 15 and 16 function differently from other balloon catheters that are used in interventional cardiology and radiology. The off-center balloon catheters of Figures 13, 15 and 16 are not dilation devices, such as those used in Percutaneous Transluminal Coronary Angioplasty. They are not intended to laterally dilate the blood vessels in which they are used. They also are not intended to stop the flow of blood and replace it with saline, as is commonly done to improve visualization during angioscopy.

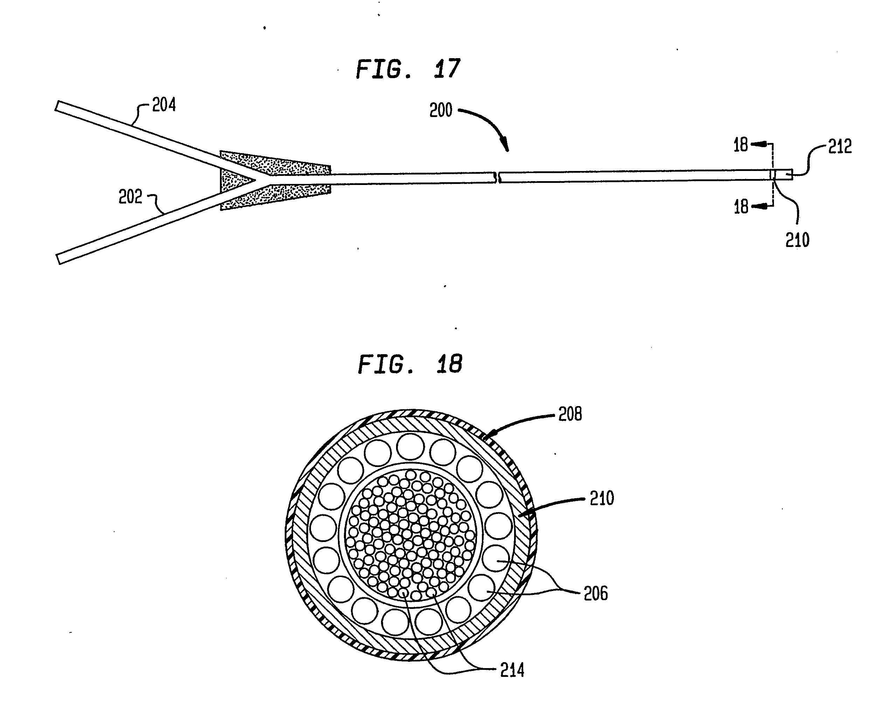

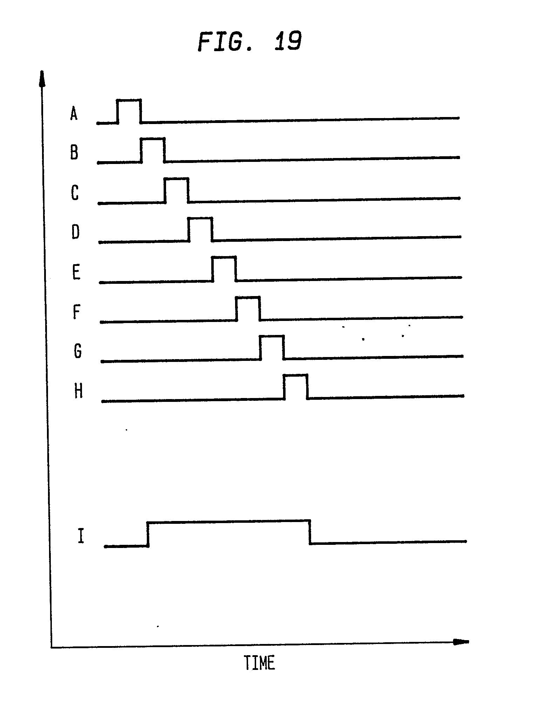

With respect to the guidewire illustrated in Figures 1 and 2, and described above, an angioscope 200 may be mounted within the hollow lumen of the coil 2. See Figures 18 and 19. The angioscope 200 may contain two types of waveguides, the first type 202 for providing a source of illuminating light and the second 204 for conducting the image from the distal end of the angioscope to an eyepiece or video camera at the proximal end thereof.

The illuminating waveguide 202 may be composed of bundle of fibers 206, each having a diameter of 30-50 micrometers. The proximal end of the illuminating waveguide 202 is connected to a light source. The imaging waveguide 204 may be composed of bundle of fibers 214 each having a diameter of 4-6 micrometers. The proximal end of the imaging waveguide 204 is connected to an eyepiece and/or a videocamera.

Typically these waveguides 202, 204 are capsulated within an outer sheath or tubing 208 having a total outside diameter of 0.013 inches or less to allow free movement of the angioscope 200 within the hollow lumen of the guidewire.

The angioscope outer sheath or tube 208 may be constructed from a material having a low coefficient of friction and constructed as thin as possible in order to provide more space that will enable an increase in the number of the imaging fibers in the imaging waveguide.

In a preferred embodiment of the angioscope 200 a gold band marker 210 is bonded to the distal end of the angioscope 200 to enable a physician to have visualization of the angioscope tip location using standard fluoroscopy equipment. The angioscope gold band 210 may be composed of a 99.9% gold alloy having a thickness of 0.001" and may be bonded at the distal end, proximal to the termination of the illuminating and viewing waveguides. The gold band 210 may be covered by the angioscope sheath or outer tube 208.

A lens 212 is bonded to the distal end of the angioscope 200 so as to conduct the images before the angioscope 200 to the viewing waveguide 202.

Figure 19 relates to a method of transmitting high energy pulsed laser beams through a substantially pure silica fiber. Figure 19 depicts a pulse that is made up of a train or a group of temporarily shifted subpulses. A high energy long pulse I is a super positioning of numerous subpulses A-H and has a shorter diffusivity of tissue. In other words, a high energy pulse duration can be stretched in time when ablating a tissue without causing undesired thermal damage to adjacent tissue due to the thermal diffusion through the tissue during the ablation process. The estimated time needed to move a high energy laser pulse from an ablation zone tissue is about one millisecond.

U.S. Patent No. 4,677,636 relates to such laser pulses, and the subject matter thereof is incorporated herein by reference.

It will be appreciated by those of ordinary skill in the art that the present invention can be embodied in other specific forms without departing from the spirit or essential characteristics thereof. The presently disclosed embodiments are therefore considered in all respects to be illustrative and not restrictive. The scope of the invention is indicated by the appended claims rather than the foregoing description, and all changes that come within the meaning and range of equivalents thereof are intended to be embraced therein.

## Claims

1. A guidewire for a catheter, comprising;
a wire coil having a distal end; and

an optical fiber having a distal end and extending axially through the centre of the wire coil, the distal end of said optical fiber being bonded to the distal end of the wire coil.

2. A guidewire according to claim 1, wherein the outer diameter of the wire coil is less than or equal to 381 μm (0.015 inches).

3. A guidewire according to claim 1 or 2, wherein the outer diameter of the optical fiber is less than or equal to 200 microns.

4. A guidewire according to any preceding claim, further comprising a plastic coating over the wire coil.

5. A guidewire according to any preceding claim, wherein the outer diameter of the wire coil tapers inwardly at the distal end thereof.

6. A catheter system, comprising:
a wire coil and an optical fiber extending axially through the centre of the wire coil;
a sleeve having two parallel lumens extending therethrough; and
an angioplasty instrument for lasing occlusions in a blood vessel extending through and bonded within one of the two lumens;
the other of the two lumens adapted to receive the wire coil.

7. A catheter system according to claim 6, further comprising a plastic coating over the wire coil.

8. A catheter system according to claim 6 or 7, wherein the outer diameter of the wire coil tapers inwardly at the distal end thereof.

9. A catheter system according to claim 6, 7 or 8, wherein the distal end of the optical fiber is bonded to the distal end of the wire coil.

10. A catheter system according to any of claims 6 to 9, wherein the angioplasty instrument includes means for conveying laser energy to an occlusion before the distal end of the angioplasty instrument.

11. A catheter system according to any of claims 6 to 10, wherein the angioplasty instrument further includes means for observing a region before the distal end of the angioplasty instrument.

12. A system for the delivery of laser energy during angioplasty, comprising:
an elongated catheter adapted to fit within a human blood vessel, said catheter being internally divided into a plurality of lumens;
a guidewire slidably disposed in one of said lumens, said guidewire comprising a flexible sheath and an optical fiber disposed within said flexible sheath;
said lumen in which said guidewire is disposed is located in the center of said catheter along its longitudinal axis; and
two lumens located radially outwardly of said center lumen and symmetrically with respect thereto, each of said two lumens containing at least one fiber-optic waveguide in a symmetrical relationship relative to said guidewire.

13. A system according to claim 12, wherein the fiber optic waveguides are made of substantially pure synthetic silica.

14. A system according to claim 12 or 13, further comprising a third lumen located radially outwardly of the centre lumen.

15. A system according to claim 12, 13 or 14, wherein said lumens located radially outwardly of said center lumen include means for conveying a saline solution from the proximal end of the catheter to the distal end of the catheter.

EP 0 355 996 A2

## FIG. 1

# FIG. 2

FIG. 3

EP 0 355 996 A2

## FIG. 4

EXCIMER LASER

2

30

28

12

51

## FIG. 5

EP 0 355 996 A2

40 38 44

42
36
34

35

33

32

12

FIG. 6A

FIG. 6B

LASER BEAM

46

48

A

F

B

LENSED
QUARTZ FIBER

47

PURE
QUARTZ
LENS

FIG. 7

## FIG. 8

6 FIBER OPTICS
TO LASER UNIT

86

GUIDE WIRE
INTRODUCTION

CONNECTOR

SALINE AND
CONTRAST MEDIA
INJECTION

98

80

9

9

## FIG. 10

96

1

94

92    91    90

100

## FIG. 9

88    80

90    1
82

88

88

FIG. 11

FIG. 12

FIG. 13

EP 0 355 996 A2

FIG. 14

FIG. 15

# FIG. 16

174'
186'
180'
172'
178'
188'
184'
1

EP 0 355 996 A2

FIG. 17

204

200

18

212

202

18

210

FIG. 18

208

210

206

214

EP 0 355 996 A2

# FIG. 19

TIME